# EUROPEAN PATENT APPLICATION

(11) **EP 3 954 784 A1**
(43) Date of publication of application: **16.02.2022**
(21) Application number: 20787907.3
(22) Date of filing: 08.04.2020
(51) Int. Cl.: C12Q 1/6886

(54) **COMPOSITION FOR DIAGNOSIS OR PROGNOSIS PREDICTION OF GLIOMA, AND METHOD FOR PROVIDING INFORMATION RELATED THERETO**

(30) Priority: 09.04.2019 KR 20190041498
(71) Applicant: Samsung Life Public Welfare Foundation, Seoul 04348 (KR)
(72) Inventor: NAM, Do-Hyun, Seoul 06351 (KR); SA, Jason Kyungha, Seoul 06351 (KR)
(74) Representative: Patentanwälte Olbricht Buchhold Keulertz
(86) International application number: PCT/KR2020/004729
(87) International publication number: WO 2020/209590

(57) **Abstract**

The present invention relates to: a composition, a biomarker panel, and a kit for diagnosis or prognosis of glioma, all of which contain an agent for detecting variants in target genes; a method of providing information related to diagnosis or prognosis of glioma; a biomarker panel for personalized medicine for glioma; and a method of providing information for personalized treatment. When genetic or protein variants are detected in at least one target gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2 according to an aspect, the diagnostic accuracy of glioma is high and sensitivity to diagnosis of glioma is also high, and thus, glioma may be effectively diagnosed. Therefore, an agent for detecting genetic variants in the target genes may be effectively used in a composition, a kit, a method of providing information for diagnosis or prognosis of glioma, and a method of providing information for personalized treatment.

## Description

### TECHNICAL FIELD

The present invention relates to: a composition, a biomarker panel, and a kit for diagnosis or prognosis of glioma, all of which contain an agent for detecting variants in target genes; a method of providing information related to diagnosis or prognosis of glioma; a biomarker panel for personalized medicine for glioma; and a method of providing information for personalized treatment.

### BACKGROUND ART

Tumors are born out of cells growing out of control in disorder and turning to a mass of abnormal cells. When such tumors are destructively proliferative, invasive, and metastatic, the tumors are considered malignant tumors. In particular, tumors, in an aspect of molecular biology, may be referred to as genetic disorders developed due to genetic variants.

Among the tumors, glioma originates in neuronal and glial cells, mostly grows invasively into nearby normal tissues, grows fast due to failure in control of cell growth, and is hard to be completely removed by surgery. In particular, gliomas fall into several different categories by histological and molecular features, but somatic mutations in IDH1/2, genomic amplification of EGFR, and co-deletion of chromosome arms 1p and 19q are checked for accurate diagnosis till now. Besides, the IDH1/2 mutations are frequently found in low-grade gliomas and secondary glioblastomas, and widely known to profoundly affect prolonged survival in glioma patients. In addition, focal amplification of EGFR is shown in about 50 % of glioblastomas. Moreover, EGFR gene amplification is known to promote cell proliferation, and thus, clinical trials for EGFR inhibitors are frequently performed for cancer treatment.

Against this backdrop, for effective diagnosis of gliomas, ilmmunohistochemistry (IHC) and fluorescence in situ hybridization (FISH) are commonly used to perform pathological diagnosis on a target mutation of IDH1 which is considered to be an essential gene for glioma proliferation, and focal amplification of EGFR, but there is a growing need for effectively diagnosing gliomas in a simpler way.

### DESCRIPTION OF EMBODIMENTS

### TECHNICAL PROBLEM

An aspect relates to a composition and a biomarker panel for diagnosis or prognosis of glioma, including an agent that detects variants in at least one gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2.

An aspect relates to a kit including an agent for detecting variants in at least one gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2.

Another aspect relates to a method of providing information on diagnosis or prognosis of glioma, the method including obtaining a nucleic acid sample from a biological sample of an individual; detecting genetic variants in at least one target gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2 from the obtained sample; and comparing and analyzing the extent of the detected genetic variants with the extent of a normal sample.

Yet another aspect relates to a biomarker panel for personalized medicine for glioma, the biomarker panel including an agent for detecting variants in at least one gene selected from the group consisting of SAMD11, KLHL21, FAM167B, HPCAL4, GPBP1 L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2.

Yet another aspect relates to a method of providing information for personalized treatment for glioma, the method including detecting genetic variants in at least one target gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2 from a biological sample isolated from an individual; and setting the individual whose genetic variants are detected in the detection results as a treatment target.

### SOLUTION TO PROBLEM

An aspect provides a composition and a biomarker panel for diagnosis or prognosis of glioma, including an agent that detects variants in at least one gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2.

Gliomas are a type of tumor that arises from glial cells in the brain or spinal cord, and are grouped by the type of cells mainly present in tumors: astrocytomas, oligodendrogliomas, and ependymomas, and compositions that allow diagnosis or prognosis through a composition of an aspect may be, for example, at least one selected from a group consisting of astrocytic tumors, oligodendroglial tumors, mixed gliomas, and ependymal tumors.

The term "diagnosis" refers to identifying the presence or characteristics of a pathological condition. For the purpose of the present invention, the diagnosis may be an identification of the onset of glioma. The term "prognosis" refers to, in relation to glioma, predictions of, for example, survival rate of individuals, the recurrence, metastasis, agent responsiveness, and resistance of the individuals after treatment. The prognosis not only allows an identification of the onset of glioma for the individuals, but also predictions of survival prognosis of the individuals by identifying the extent of variants in at least one gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2 of an aspect from individual samples.

Individuals with gliomas are known to be closely related to co-deletion of the short arm (1p) of chromosome 1 and the long arm (19q) of chromosome 19, and out of the individuals with gliomas, for individuals having the co-deletion of 1p and 19q, it is widely known that anticancer agent sensitivity is closely related to an increase in survival rate, and thus it is known that the co-deletion of 1p and 19q allows effective prognosis of individuals. Accordingly, it is confirmed that an agent of an aspect effectively detects the co-deletion of 1p and 19q to identify 20 target genes for accurate diagnosis and prognosis of gliomas, and through the detection of the variant extent of the 20 genes, individuals with gliomas and their prognosis are predicted with high accuracy and sensitivity.

Variants of the 20 target genes identified in the above aspect are highly expressed in the blood of glioma patients compared to individuals without gliomas, and thus may be used for diagnosing individuals with gliomas, which are particularly difficult to diagnose clinically.

SAMD11 is known to be present at chr1:860235-879558 on chromosome, and is represented by Ensembl ID NO: ENSG00000187634. KLHL21 is known to be present at chr1:6653400-6674692 on chromosome, and represented by Ensembl ID NO: ENSG00000162413. FAM167B is known to be present at chr1:32712793-32714227 on chromosome, and represented by Ensembl ID NO: ENSG00000183615. HPCAL4 is known to be present at chr1:40148183-40157407 on chromosome, and represented by Ensembl ID NO: ENSG00000116983. GPBP1L1 is known to be present at chr1:46093903-46152327 on chromosome, and represented by Ensembl ID NO: ENSG00000159592. LPHN2 is known to be present at chr1:81771820-82458145 on chromosome, and represented by Ensembl ID NO: ENSG00000117114. GPR88 is known to be present at chr1:101003668-101007608 on chromosome, and represented by Ensembl ID NO: ENSG00000181656. ZNF599 is known to be present at chr19:35249914-35264159 on chromosome, and represented by Ensembl ID NO: ENSG00000153896. C190RF33 is known to be present at chr19:38794776-38795671 on chromosome, and represented by Ensembl ID NO: ENSG00000167644. B9D2 is known to be present at chr19:41860580-41870103 on chromosome, and represented by Ensembl ID NO: ENSG00000123810. BCAM is known to be present at chr19:45312291-45324698 on chromosome, and represented by Ensembl ID NO: ENSG00000187244. CABP5 is known to be present at chr19:48533789-48547336 on chromosome, and represented by Ensembl ID NO: ENSG00000105507. SIGLEC11 is known to be present at chr19:50453202-50464454 on chromosome, and represented by Ensembl ID NO: ENSG00000161640. ERVV-2 is known to be present at chr19:53547966-53554405 on chromosome, and represented by Ensembl ID NO: ENSG00000268964. ZNF865 is known to be present at chr19:56116746-56129932 on chromosome, and represented by Ensembl ID NO: ENSG00000261221. MZF1 is known to be present at chr19:59073414-59084967 on chromosome, and represented by Ensembl ID NO: ENSG00000099326. MRTO4 is known to be present at chr1:19578008-19585349 on chromosome, and represented by Ensembl ID NO: ENSG00000053372. LRIG2 is known to be present at chr1:113615767-113669847 on chromosome, and represented by Ensembl ID NO: ENSG00000198799. BSND is known to be present at chr1:55464581-55474326 on chromosome, and represented by Ensembl ID NO: ENSG00000162399. SLC30A2 is known to be present at chr1:26365626-26372654 on chromosome, and represented by Ensembl ID NO: ENSG00000158014.

In an aspect, an agent included in a composition may be 1) single nucleotide variant, 2) deletion or insertion of a base sequence region of 1 to 50 nucleotides, 3) copy number variant, or 4) a combination of two or more selected from 1) to 3) above.

The 'variant' may include an alteration of a base, a nucleotide, a polynucleotide, or a nucleic acid. The variant may include substitution, insertion, deletion, or insertion and deletion (called also as InDel) of a base, a nucleotide, a polynucleotide, or a nucleic acid. Substitution refers to an alteration in which a base, nucleotide, polynucleotide, or nucleic acid is replaced with another base, nucleotide, polynucleotide, or nucleic acid. Insertion refers to an alteration in which another base, nucleotide, polynucleotide, or nucleic acid is added. Deletion refers to an alteration in which a base, nucleotide, polynucleotide, or nucleic acid is removed.

Single nucleotide sequence variant or single nucleotide variant (SNV) refers to an alteration or variant in a sequence showing a difference of a single base or nucleotide in the genome. A single nucleotide variant and a single nucleotide polymorphism (SNP, hereinafter referred to as 'SNP') may be used interchangeably. SNPs refer to base or nucleotide sequence diversity occurring when a single base or nucleotide (adenine (nucleotide A), thymine (nucleotide T), cytosine (nucleotide C) or guanine (nucleotide G)) in the genome differs between members of a species or paired chromosomes in an individual. SNPs may be a sequence of two or more alleles or nucleotides present in a small fraction of the population (frequency ≥ 1 %, or ≥ 5%). SNPs are the most abundant genetic polymorphisms in the human genome, and may cause large differences in each individual depending on the location of the SNPs genetically. For example, when an SNP is present in a protein-coding region, the SNP may affect the structure of protein to alter functions of the protein, and may end up causing diseases. When an SNP is present in a non-protein-coding region, that is, present in a promoter or an intron, there may be a difference in expression of protein for each to increase or decrease the overall activity of the protein, and thus abnormal protein may be expressed through alternative splicing.

The copy number variant refers to a variant section having a length of 1 kb or more, which is different in copy number from reference sequence, and a method of measuring the copy number variant is, for example, fluorescence in situ hybridization (FISH), chromatin immune precipitation (ChIP), and next-generation sequencing (NGS), but is not limited thereto. In a specific embodiment, when the copy number variant of genes including SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2 is detected, co-deletion of 1p and 19q of chromosomes 1 and 19 may be caused to effectively detect gliomas.

The term "biomarker panel" refers to any combination of biomarkers for diagnosis of gliomas, and the combination may refer to an entire set or any subset or subcombination thereof. That is, the biomarker panel may refer to a set of biomarkers, and any form of the biomarker that is measured. Thus, when RPL23 is part of a biomarker panel, then either RPL23 mRNA or protein, for example, may be considered to be part of the panel. While individual biomarkers are useful as diagnostics, combination of biomarkers may sometimes provide greater value in determining a particular status than single biomarkers alone. Specifically, the detection of a plurality of biomarkers in a sample may increase the sensitivity and/or specificity of the test. Thus, in a specific embodiment, a biomarker panel may include 1, 2, 3, 4, 5, 6, 7, 8, 9, 10, 11, 12, 13, 14, 15, 16, 17, 18, 19, 20, or more types of biomarkers. In another specific embodiment, the biomarker panel consists of a minimum number of biomarkers to generate a maximum amount of information. Accordingly, in various specific embodiments, the biomarker panel may consist of 1, 2, 3, 4, 5, 6, 7, 8, 9, 10, 11, 12, 13, 14, 15, 16, 17, 18, 19, 20, or more types of biomarkers. Where a biomarker panel consists of "a set of biomarkers", no biomarkers other than those of the set are present. In a specific embodiment, the biomarker panel consists of 1 biomarker disclosed herein. In a specific embodiment, the biomarker panel consists of 2 biomarkers disclosed herein. In a specific embodiment, the biomarker panel consists of 3 biomarkers disclosed herein. In a specific embodiment, the biomarker panel consists of 4 biomarkers disclosed herein. In a specific embodiment, the biomarker panel consists of 5 biomarkers disclosed herein. In a specific embodiment, the biomarker panel consists of 6 biomarkers disclosed herein. In a specific embodiment, the biomarker panel consists of 7 biomarkers disclosed herein. In a specific embodiment, the biomarker panel consists of 8 biomarkers disclosed herein. In a specific embodiment, the biomarker panel consists of 9 biomarkers disclosed herein. In a specific embodiment, the biomarker panel consists of 10 biomarkers disclosed herein. In a specific embodiment, the biomarker panel consists of 11 biomarkers disclosed herein. In a specific embodiment, the biomarker panel consists of 12 biomarkers disclosed herein. In a specific embodiment, the biomarker panel consists of 13 biomarkers disclosed herein. In a specific embodiment, the biomarker panel consists of 14 biomarkers disclosed herein. In a specific embodiment, the biomarker panel consists of 15 biomarkers disclosed herein. In a specific embodiment, the biomarker panel consists of 16 biomarkers disclosed herein. In a specific embodiment, the biomarker panel consists of 17 biomarkers disclosed herein. In a specific embodiment, the biomarker panel consists of 18 biomarkers disclosed herein. In a specific embodiment, the biomarker panel consists of 19 biomarkers disclosed herein. In a specific embodiment, the biomarker panel consists of 20 biomarkers disclosed herein. The biomarkers of the present invention show a statistically significant difference in glioma diagnosis and prognosis. In a specific embodiment, diagnostic tests that use these biomarkers alone or in combination show a sensitivity and specificity of at least about 85 %, at least about 90 %, at least about 95 %, at least about 98 %, and about 100 %.

The biomarker may be obtained from transcript data of cells.

In an aspect, the agent for detecting genetic variants may include a primer pair, a probe, or an antisense nucleotide. Specifically, the agent may be one for measuring the variant extent of the biomarker genes, and may be a primer pair, a probe, or an antisense nucleotide that specifically binds to the genes. In a specific embodiment, the biopanel may include at least two primer pairs, probes, or antisense nucleotides, and each primer pair, probe, or antisense nucleotide may specifically bind to SAMD11, KLHL21, FAM167B, HPCAL4, GPBP1L1, LPHN2, GPR88 , ZNF599, C19ORF33, B9D2, BCAM, CABP5, SIGLEC11, ERVV-2, ZNF865, MZF1, MRTO4, LRIG2, BSND, and SLC30A2.

In particular, the primer may have a feature of amplifying a sequence such that an amplified PCR product may obtain a comparison sequence having an optimal size to effectively perform diagnosis and prognosis of gliomas, using next-generation sequencer (NGS).

The primer, probe, or antisense nucleotide may be a polynucleotide labeled with a detectable label. The detectable label is a label material capable of generating detectable signals, and may be a label material capable of generating detectable signals, including a fluorescent material, for example, substances such as Cy3 and Cy5. The detectable label may confirm the hybridization result of nucleic acids.

The term primer refers to a single-stranded oligonucleotide capable of serving as an initiation point in the polymerization of nucleotides through polymerase. For example, the primer may be a single-stranded oligonucleotide capable of serving as an initiation point for template-directed DNA synthesis at a suitable temperature, in a suitable buffer, and under suitable conditions, i.e., in the presence of four different nucleoside triphosphates and polymerase. A suitable length of a primer may depend on a variety of factors, such as temperature and the use of the primer. The primer may have a length of 5 to 100 nt, 5 to 70 nt, 10 to 50 nt, or 15 to 30 nt. For example, when a primer has a shorter length, a hybridization complex which is sufficiently stable with a template at a low annealing temperature may be formed.

The primer may further include a nucleotide analogue such as phosphorothioate, alkylphosphorothioate, peptide nucleic acid, or an intercalating agent. In addition, the primer may further include a label material that emits fluorescence, phosphorescence, or radioactivity. The fluorescent label material may be VIC, NED, FAM, PET, or a combination thereof. The label material may be labeled at the 5' -end of the polynucleotide. In addition, a radiolabeled material may be incorporated into an amplification product through a PCR reaction using a polymerase chain reaction (PCR, hereinafter referred to as 'PCR') solution to which radioisotope such as 32P or 35S is added.

The term probe refers to a polynucleotide capable of sequence-specific binding to a complementary polynucleotide strand. The probe may have a length of 5 to 100 nt, 10 to 90 nt, 15 to 80 nt, 20 to 70 nt, or 30 to 50 nt. The probe may be used in a hybridization method, for example, microarray, Southern blotting, dynamic allele-specific hybridization, a method using a DNA chip, and the like. Microarray is used as is generally understood in the art, and for example, a probe or a group of probes may be immobilized in a plurality of separated regions on a substrate. The substrate is a suitably rigid or semi-rigid support, and may include, for example, membranes, filters, chips, slides, wafers, fibers, magnetic or non-magnetic beads, gels, tubing, plates, polymers, microparticles, and capillaries. The probe or a probe complementary thereto may be hybridized with nucleic acids obtained from an individual and used in a method of measuring the extent of hybridization obtained therefrom.

An aspect provides a kit including an agent for detecting variants in at least one gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2.

The kit may be a kit for predicting genetic variants in a target gene. The kit is used as is generally understood in the art. The kit may include, for example, the polynucleotide as described above and items required for specific use thereof. The kit may include, together with the polynucleotide as described above, a reagent required for a method of using the polynucleotide.

The kit of an aspect may include a reagent for performing an amplification reaction, and may include a thermostable DNA polymerase, dNTPs, buffers, and the like. In addition, the kit of the present invention may further include a user's guide describing optimal conditions for performing reactions. The user's guide is a printout describing how to use the kit, for example, how to prepare a PCR buffer, and suggested reaction conditions. The user's guide includes a brochure in the form of a pamphlet or leaflet, a label attached to the kit, and instructions on the surface of a package containing the kit. In addition, the user's guide includes information published or provided through electronic media such as the Internet.

Another aspect provides a method of providing information on diagnosis or prognosis of glioma, the method including obtaining a nucleic acid sample from a biological sample of an individual; detecting genetic variants in at least one target gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2 from the obtained sample; and comparing and analyzing the extent of the detected genetic variants with the extent of a normal sample.

The method includes obtaining a nucleic acid sample from a biological sample of an individual.

The individual may be mammals including humans.

The biological sample refers to a sample obtained from a living organism. The biological sample may be, for example, at least one selected from the group consisting of blood, plasma, serum, urine, and saliva of a patient.

A conventional DNA isolation method of detecting genetic variants in a target gene from the biological sample may be further included and performed. For example, in the isolation method, a target nucleic acid may be amplified through polymerase chain reaction (PCR), ligase chain reaction (LCR), transcription amplification, or realtime-nucleic acid sequence based amplification (NASBA), purified, and obtained.

The analyzing of the genetic variants, based on the presence or absence of specific variants in the target genes, may identify whether an individual or a subject is at risk of contracting glioma or a patient having glioma.

The detecting of the genetic variants in the target genes may be, for example, performed through a next-generation sequencer platform, which may be whole-genome sequencing, whole-exome sequencing, or target gene panel sequencing, but is not limited thereto.

In addition, in the comparing and analyzing of the extent of the detected genetic variants with the extent of normal samples, the analysis of the extent of the genetic variants may be to identify the presence of co-deletion of gene chromosomes 1 p and 19q.

The providing of information on diagnosis or prognostic prediction of gliomas in the individuals may be to predict the relative risk for co-deletion of gene chromosomes 1p and 19q, which are highly associated with glioma expression to measure the genetic variant extent of at least one gene selected from the group consisting of SLC30A2 SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2. For example, the risk may be for predicting or diagnosing whether the likelihood of developing glioma is increased when the variant extent of the target genes is high compared to the group having the reference genome sequence. When the variant in the target genes is 1 or more, 2 or more, 3 or more, 4 or more, 5 or more, 6 or more, 7 or more, 8 or more, 9 or more, or 10 or more, 11 or more, 12 or more, 13 or more, 14 or more, 15 or more, 16 or more, 17 or more, 18 or more, 19 or more, or 20 or more, it is believed to have a high risk of developing glioma, which is highly related thereto due to a high possibility of co-deletion of gene chromosomes 1p and 19q, and through this, the accuracy of the diagnosis or prognostic prediction of glioma may be increased. In a specific embodiment, when copy number variants were measured in all 20 target genes, it was confirmed that the co-deletion of genome chromosomes 1p and 19q was found, thereby effectively diagnosing glioma.

In an aspect, the comparing and analyzing of the extent of the detected genetic variants with the extent of normal samples may include determining an individual exhibiting co-deletion of genome chromosomes 1p and 19q as an individual with glioma exhibiting co-deletion of chromosomes 1p and 19q when the copy number variants are found in all 20 target genes. In addition, when an individual exhibits co-deletion of chromosomes 1p and 19q, it is possible to determine whether or not patients have a high survival rate, and thus, the comparing and analyzing of the extent of the detected genetic variants with the extent of normal samples may further include predicting a good prognosis for glioma patients because the glioma patients have a high survival rate when the copy number variants are shown in all 20 target genes, and the individual is determined to be an individual with glioma showing co-deletion of chromosomes 1p and 19q.

In the method above, the detecting of genetic variants in the genes is a method of determining a nucleotide or base sequence, and may be sequencing, for example, whole-genome sequencing, whole-exome sequencing, or target gene panel sequencing, and may be performed through at least one method selected from the group consisting of microarray hybridization, allele specific PCR, dynamic allele-specific hybridization, PCR extension analysis, PCR-single strand conformation polymorphism (PCR-SSCP), and TaqMan. The detecting of genetic variants in the genes may use the composition or the kit.

Yet another aspect provides a biomarker panel for personalized medicine for glioma, the biomarker panel including an agent for detecting variants in at least one gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2.

Yet another aspect provides a method of providing information for personalized treatment for glioma, the method including detecting genetic variants in at least one target gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2 from a biological sample isolated from an individual; and setting the individual whose genetic variants are detected in the detection results as a treatment target.

As described above, the biomarker gene panel according to a specific embodiment is based on an individual patient's oncogene, and thus the therapeutic effect may be enhanced for gliomas through customized gene therapy. In addition, in the gene panel, the copy number variant is a target, and thus, it may effectively perform target treatment on glioma, which is a variety of diseases caused by the genetic variants.

In an embodiment, when copy number variants were measured in all 20 target genes, it was confirmed that the co-deletion of genome chromosomes 1p and 19q was found, thereby effectively diagnosing glioma. When the co-deletion of chromosomes 1p and 19q is found, patients with gliomas have a higher survival rate, and thus, when copy number variants are found in all 20 target genes, it may be predicted that the prognosis of the glioma patients is good.

It is known in the art that when the co-deletion of chromosomes 1p and 19q is found, chemosensitivity and survival rate to a specific anticancer agent therapy may change. For example, in oligodendroglioma, it is known that patients with the co-deletion of chromosomes 1p and 19q show different responses to chemotherapy, also it is known that patients with the co-deletion of chromosomes 1p and 19q have a higher survival rate when radiation therapy is applied in combination with procarbazine / lomustine / vincristine chemotherapy (PCV) compared to radiation therapy alone, and it is known that oligodendrocyte patients with the co-deletion of chromosomes 1p and 19q have excellent prognosis due to anticancer agent sensitivity, and thus, with an individual showing genetic variants for 20 target genes according to an aspect, personalized treatment may be performed on individuals with or without co-deletion of chromosomes 1p and 19q, and targeted therapy may also be applicable.

The method of providing information in an embodiment includes detecting mutations of the defined gene panel from a biological sample isolated from an individual. Specifically, the individual refers to a subject for predicting the risk of developing glioma due to genetic variants. The individual may include a vertebrate, a mammal, or a human (Homo sapiens). For example, the human may be Korean. In addition, the biological sample may be tissue, cell, whole blood, serum, plasma, saliva, sputum, cerebrospinal fluid, or urine. The detecting of mutations of the defined gene panel above may be performed by isolating nucleic acids from the biological sample and then measuring copy number variants, and the method of isolating nucleic acids and measuring copy number variants is known in the art. The method of isolating nucleic acids may be performed by for example, directly isolating DNA from the biological sample or amplifying a specific region through a nucleic acid amplification method such as PCR. The isolated nucleic acid sample includes not only purely isolated nucleic acids, but also crudely isolated nucleic acids, for example, cell debris containing nucleic acids. The nucleic acid amplification methods include PCR, ligase chain reaction (LCR), transcription amplification, self-sustained sequence replication, and nucleic acid sequence based amplification (NASBA). The isolated nucleic acid may be DNA or RNA. The DNA may be genomic DNA, cDNA, or recombinant DNA. The RNA may be mRNA. In addition, in the method of determining the position of mutations, for example, nucleotide at the position of mutations may be directly determined through a known nucleotide sequencing method of nucleic acids. The method of sequencing nucleotides may include a Sanger (or dideoxy) sequencing method or a Maxam-Gilbert (chemical cleavage) method. In addition, nucleotides at the position of mutations may be determined by hybridizing a probe including the sequence of the position of mutations with target polynucleotides, and analyzing hybridization results. The extent of hybridization may be confirmed, for example, by labeling a target nucleic acid with a detectable label, and detecting the hybridized target nucleic acid, or by an electrical method or the like. In addition, a single base primer extension (SBE) method may be used.

Unless otherwise defined, all the technical terms used herein correspond to the meanings as generally understood by those skilled in the art. In addition, preferred methods or samples are described herein, but those similar or equivalent thereto are incorporated in the scope of the invention. The contents of all the publications disclosed as references herein are incorporated herein by reference in their entirety.

### ADVANTAGEOUS EFFECTS OF DISCLOSURE

When genetic or protein variants are detected in at least one target gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2 according to an aspect, the diagnostic accuracy of glioma is high and sensitivity to diagnosis of glioma is also high, and thus glioma may be effectively diagnosed. Therefore, an agent for detecting genetic variants in the target genes may be effectively used in a composition, a kit, and a method of providing information for diagnosis or prognosis of glioma, and a method of providing information for personalized treatment.

### BRIEF DESCRIPTION OF THE DRAWINGS

FIG. 1 is a view showing evaluation results of comparing the sensitivity of GliomaSCAN with WES data.
FIG. 2 is a view showing results of confirming variant profiles of treatment target genes from 46 specimens.
FIG. 3 is a view confirming information on variants frequently found in gliomas affecting individual gliomas.
FIG. 4A is a view confirming detection accuracy of copy number loss of 1p and 19q for 20 selected target genes.
FIG. 4B is a view showing results of verifying the effectiveness of the selected 20 target genes by comparing GliomaSCAN and FISH data.
FIG. 4C is a view showing results of confirming the comparison of CNAs at the chromosomal level from co-deletion of 1p and 19q and wild-type specimens of 1p and 19q.

### MODE OF DISCLOSURE

Hereinafter, the present invention will be described in detail through Experimental Examples and Examples. It would be obvious to those skilled in the art that the following examples are intended to be more concretely illustrative and the scope of the present invention as set forth in the appended claims is not limited to or by the examples.

### Experimental Examples - Selection of mutations for selecting biomarkers for diagnosis of glioma

### <Experimental Example 1> Collection and analysis of subjects' tumor specimens for diagnosis of glioma

Tumor specimens from cancer patients for accurate diagnosis of gliomas were collected under the approval of the Bioethics Committee with consent from some patients who visited Samsung Medical Center from January 2014 to August 2017. The IRB of Samsung Medical Center (IRC#: 2010-04-004) has approved this study. The tumor specimens were diagnosed. Specimens of cancer patients for the diagnosis were selected based on the criteria: (i) possibility of a patient being enrolled in a clinical trial when a viable mutation is found; (ii) possibility of a patient's specimen being stored in the pathology department as a tumor fraction in sufficient quantity. The tumor specimens were processed without binding together with normal tissues.

The tumor specimens collected through the method described above were sequenced with GliomaSCAN, which is a target sequencing panel designed by Samsung Medical Center. 232 target genes related to glioma (target cancer) therapy that has been approved by Korea MFDS and US FDA, or that has been reported to be useful as cancer therapy in Catalogue of Somatic Mutations in Cancer (COSMIC, public database) and literature were selected through the panel, and the genes from the specimens were analyzed through the panel.

The cancer specimens were analyzed through the panel based on specificity, limit of detection, PPV and NPV from a set of gene pool consisting of HD753 (Horizon, Inc. USA), NA12878, and 10 cell lines (NA07014, NA10840, NA18488, NA18511, NA18595, NA18867, NA18924, NA18957, NA19108, and NA19114). For the HD753 cell line, sensitivity was calculated by comparing known variants and variant allele frequency (VAF) measured through the panel with projected values of single nucleotide variant (SNV), insertion and deletion (InDel), and gene copy number variant (CNV). For the NA12878 cell line, genotype of SNV in the panel was compared with NA12878 in a coding region to calculate sensitivity, specificity, PPV and NPV. In addition, to predict limit of detection and sensitivity, the measured VAFs of SNVs in the panel were compared to the projected VAFs in the set of gene pool.

### <Experimental Example 2> Comparison with whole exome sequencing for cross-validation of collected glioma specimens

Tumor specimens and clinical information from studies approved by the institutional review board of Samsung Medical Center, Catholic Medical Center of Catholic University of Korea, and Seoul National University Hospital were obtained from brain tumor patients who underwent tumor removal surgery at the institutions, and histological diagnosis of tumors was performed after obtaining informed consent from all patients. For genetic analysis, a portion of the tumor specimens was snap-frozen and preserved in liquid nitrogen before use. Genomic DNA was extracted using a DNeasy kit (Qiagen). Target sequencing was performed on 46 specimens, of which 28 specimens were simultaneously subjected to Whole-Exome Sequencing (WES). 52 specimens and 45 specimens were subjected to WES and target sequencing to identify chromosomes 1p and 19q.

### <Experimental Example 3> Analysis of target sequencing

Sequence gene reads in FASTQ files were mapped to human genome assembly (hg19), using Burrows-Wheeler Aligner version 0.6.2 or mutation calling. MuTect and SomaticlndelDetector were used to perform high-confidence prediction of somatic mutations from tumor and normal tissue pairs. A Variant Effect Predictor (VEP) version was used to annotate the predicted somatic mutations with potential functional consequences and other relevant information. Significant mutations were selected when the total gene reads were 20 or more, or the VAF was greater than 0.05.

In addition, gene copy number was measured using ngCGH python package 0.4.4 version. Normal WES data matched with patients were used as a criterion for predicting fold change in the copy number of tumors. Genomic amplification and deletion were determined on a log2 scale of 0.585 or more and a log2 scale of -0.5 or less, respectively.

### <Experimental Example 4> Statistical analysis of target sequencing

The VAF correlation between WES and target sequence was calculated by Pearson. For evaluation of 1p/19q status using target sequencing analysis, the copy number status of genes located on chromosomes 1p and 19q were used to investigate using a receiver operating characteristic curve (ROC) and to measure in trapezoidal. For visualization changes, Oncoprint plots were used through R package and composite heatmaps. A two-sided Fisher's exact test was used to compare somatic mutations of IDH1 with focal amplification of EGFR between GliomaSCAN and FISH or IHC.

### Example 1 Validation of effectiveness of panels from tumor cell lines

### 1.1 Validation of effectiveness of panel from HD753 cell line

A total of 15 variants out of 18 variants were identified from the target region through analysis using GliomaSCAN. Compared to the variants identified from a mutation measurement panel, all the 15 variants, including 3 SNVs, 2 SNVs having high GC content, 1 SNV having low GC content, 1 long insertion, 1 long deletion, and 3 short deletions were detected. There was a high correlation between expected and measured allele frequencies (r² = 0.9356). As a result, the sensitivity of SNV, InDel, and CNV was found to be 100 %.

### 1.2 Reference sequencing from NA12878 cell line

Genotype was thoroughly analyzed and the NA12878 cell line was used as a reference sequence to estimate the sensitivity and specificity of SNVs to 4,330 target regions (1,269 kb) in the panel. When the SNVs identified in the panel and the genotype of NA12878 (ftp://ftptrace.ncbi.nlm.nih.gov/giab/ftp/release/NA12878_HG001/latest/GRCh37/) were compared, both SNVs identified were consistent. In addition, 846,058 reference genotypes of the target regions were compared and found to be consistent with the genotypes identified in the panel.

### 1.3 Validation of effectiveness of panels by measuring genetic variant detection limits and sensitivity

Limit of detection (LOD) and detection sensitivity were measured using the set of gene pool (expected variant allelic frequency (VAF) range: 4 % to 100 %, variant number in the gene set for panel validation: 689). When the effectiveness of panels was evaluated using the panel, the sensitivity and correlation ratio (r2) were 99.2 % and 0.9856, respectively, confirming that it was possible to measure the variants of the gene set quite accurately. It was confirmed that 135 out of 136 variants were detected (99.26 %) in the low expected VAF range (4.1 % to 5 %), taken together, confirming that the limit of detection of genetic variants according to the panel was 5 % or more.

### Example 2 Validation of effectiveness of panels by comparison of variant allele frequencies between target sequencing panel and whole exome sequencing

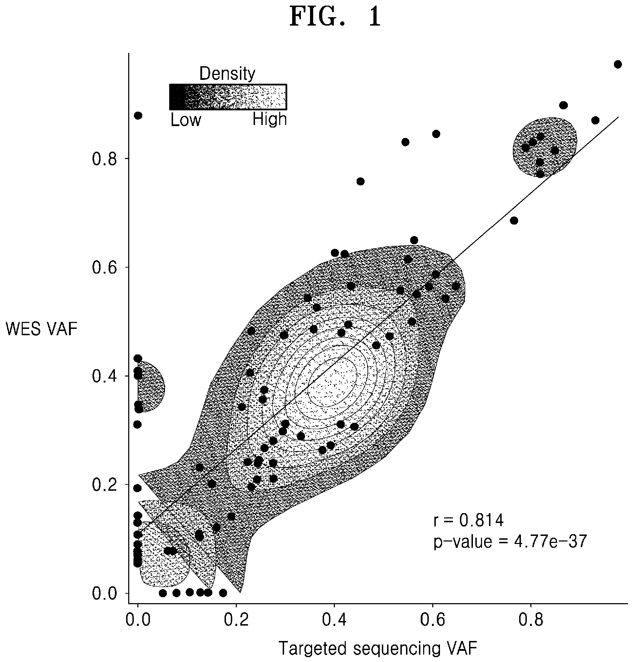

An experiment was performed to compare the variant allele frequencies of somatic mutations obtained from the target panel with WES to identify the accuracy of the target sequencing panel. 28 specimens of tumor tissue matched with DNA extracted from normal blood were exposed to WES and the target sequencing panel. Thresholds of significant mutations from SNVs, insertions, or deletions were set at depth range ≥ 20 and VAF ≥ 5 %. VAFs of all somatic mutations measured in WES and the target sequencing analysis were compared and are shown in FIG. 1.

FIG. 1 confirms that a total of 118 genomic variants were detected in both sequencing panels, and 24 or 9 variants were detected in WES or the target sequencing analysis, respectively. It is believed that when DNA samples subjected to the target sequencing panel were compared to tumor fragments subjected to WES, private mutations having relatively low VAF were measured because glioblastoma exhibits excessive intratumoral heterogeneity as derived from different sites of the same tumor. The low VAF confirms the presence of sub-clonal mutations, such as when tumor cells proliferate, and confirms that various private mutations may be obtained. Conversely, a small number of private mutations having relatively high VAF were identified in the WES platform. By analyzing each private mutation, it was confirmed that the genetic variants were real germ cell mutations as detected simultaneously in matched normal blood and not found in the target sequencing panel. The absence of the WES blood panel is mainly caused by a significant difference in sequencing depth between the WES and the target sequencing panel. In particular, the range of GliomaSCAN is measured to be about 800 X, which is much higher than that of standard WES, 200X, but in terms of somatic mutation frequency (r = 0.814, P-value = 4.77e-37; Fisher exact test), it was confirmed that WES and the target sequencing panel showed a high correlation. Therefore, it was confirmed that the target sequencing panel confirmed in the Experimental Example was capable of accurately identifying and measuring potential somatic variants at a high level of confidence compared to the previously established WES platform.

### Example 3 Identification of genetic variants in biomarker genes capable of diagnosing glioblastoma

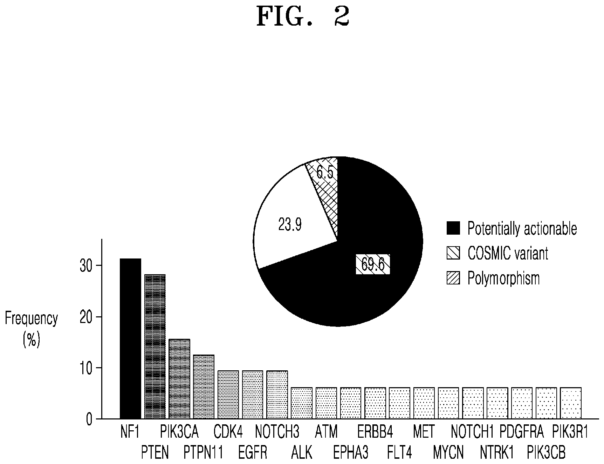

An experiment was performed to confirm genetic variants as a biomarker capable of easily diagnosing glioblastoma, which used to be confirmed only through conventional clinical judgment. As confirmed in Experimental Example, clinically usable mutations that may effectively detect glioblastoma across genes out of 94 genes known to see the therapeutic effects identified in Experimental Example were systematically evaluated, and results of the evaluation are shown in FIG. 2.

As shown in FIG. 2, 32 out of 46 patients (69.6 %) were confirmed to have at least one somatic mutation in the genes that are clinically feasible. In addition, it was confirmed that 11 out of 46 patients (23.9 %) had variants previously identified in COSMIC, among which mutations in the NF1 gene were the most common mutations (31.3 %), and mutations in the PTEN, PIK3CA, and PTPN11 genes were 28.1 %, 15.6 %, and 12.5 %, respectively, as the second most common mutations after the NF1 gene. In addition, as shown in FIG. 2, it was confirmed that mutations of 19 genes were found in at least two or more patients. For recurrently identified mutations, the number of somatic mutations in the genes varied from 2 to 81 in 32 specimens. Collectively, it was confirmed that genes that are useful as predictive indicators of potential treatment response identified in 43 out of 46 patients (93.5 % of specimens) had at least one genetic variant.

### Example 4 Identification of frequently identified somatic variants in gliomas

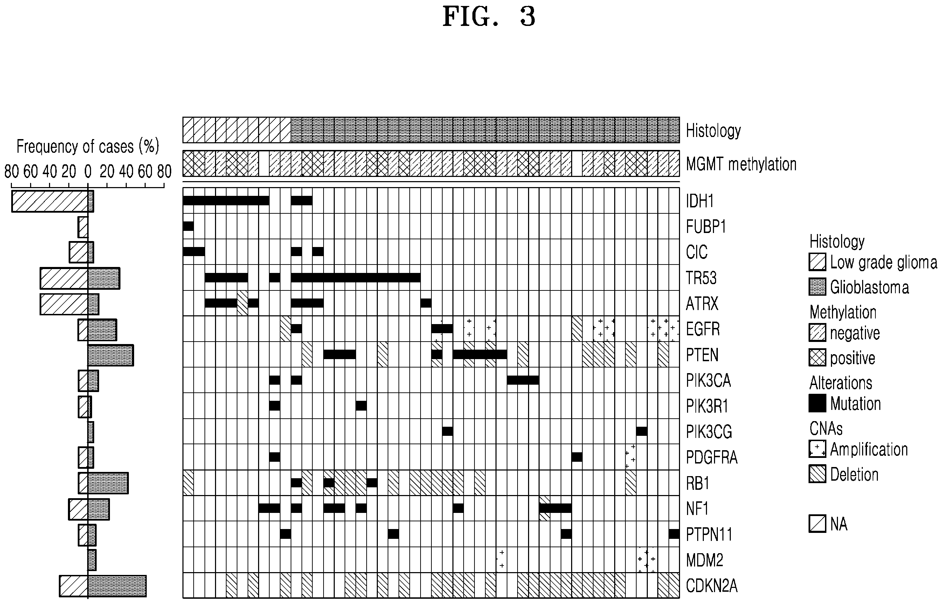

Gliomas may be effectively diagnosed through mutations frequently identified in advanced gliomas, using target sequencing panels, and accordingly, an experiment was performed to analyze the mutations. Single nucleotide variants, short insertions and deletions were analyzed in 10 specimens of low-grade glioma (LGG) and 36 specimens of glioblastomas (GBM). It is critical to identify whether such variants are present in gliomas because the genetic variants regulate key carcinogenic pathways that are often out of control in glioblastomas, and previous studies have shown that TP53 mutations are known to be found at early stages of tumor progression across multiple tumor types. Therefore, the WHO reclassification states that not only IDH1/IDH2 variants but also TP53 variants are required to be considered for glioma diagnosis, and in particular, it is essential to identify the TP53 variants since co-deletion of chromosome arms 1p and 19q is not consistent with the TP53 mutations. In addition, glioblastoma may be classified into four separate molecular subtypes according to genetic variants accompanying transcript expression, and the molecular subtypes are known to be classified based on somatic variants in cancer-inducing genes such as CNA, EGFR, NF1, and PDGFRA. Mutations frequently identified in the genes considerably differ between molecular subtypes having distinct biological characteristics, and thus, it is important to select a target gene candidate group that may be used as a biomarker for accurately diagnosing glioma. The appearance rates of CNAs were confirmed in 9 genes (TP53, ATRX, EGFR, PTEN, PDGFRA, RB1, NF1, MDM2, and CDKN2A) among the target gene candidate group, and are shown in FIG. 3.

As confirmed in FIG. 3, while the expression rates of mutations in IDH1 and ATRX genes are remarkable, several important gene variants including focal amplification of EGFR and gene deletion of PTEN were identified in glioma, confirming that the findings are consistent with the previously known fact that an increase in chromosome 7 and deletion of chromosome 10 induce significant oncogenesis of glioblastoma. In addition, it was confirmed that variants in the IDH1 gene and ATRX gene were found at a frequency of 8 and 4 out of 10 variants in the low-grade gliomas. It was confirmed that amino acids were changed to R132H, except for variants in which a stop codon was obtained as R20 * in the IDH1 protein among the IDH gene variants.

### Example 5 Determination of biomarkers for prognosis and diagnosis of glioma

An experiment was performed to determine a biomarker for measuring co-deletion of chromosomes 1p/19q for diagnosis and more accurate prognosis of glioma. Target sequencing analysis data were performed with GliomaSCAN which was confirmed to be effective in Experimental Example, and the target sequencing analysis data were verified to be effective compared to current standard methods once, and accordingly, an experiment to determine a biomarker capable of measuring co-deletion of genes 1p/19q with validity was performed as well.

It was confirmed that the target sequence analysis by GliomaSCAN confirmed in Experimental Example was consistent with the results of Fisher's exact test of EGFR amplification performed through an independently confirmed FISH experiment as both share a P-value of 2.06e-04, and it was confirmed that even compared with the IHC method, which is the current standard measurement method for detecting IDH1 mutations in gliomas, the target sequence analysis by GliomaSCAN showed high consistency (P-value was 6.68e-07).

Accordingly, from the 232 target genes of Experimental Example 1 to determine a biomarker for effectively identifying co-deletion of chromosomes 1p/19q, which is confirmed to be effective as above, 20 genes in which the co-deletion of chromosomes 1p/19q was effectively identified were selected, and the genes are shown in Table 1 below.

**[Table 1]**

| SAMD1 1 | KLHL2 1 | FAM167 B | HPCAL 4 | GPBP1 L1 | LPHN 2 | GPR8 8 | ZNF59 9 | C19OR F33 | B9D2 |
|---|---|---|---|---|---|---|---|---|---|
| BCAM | CABP5 | SIGLEC 11 | ERVV-2 | ZNF865 | MZF1 | MRTO 4 | LRIG2 | BSND | SLC30 A2 |

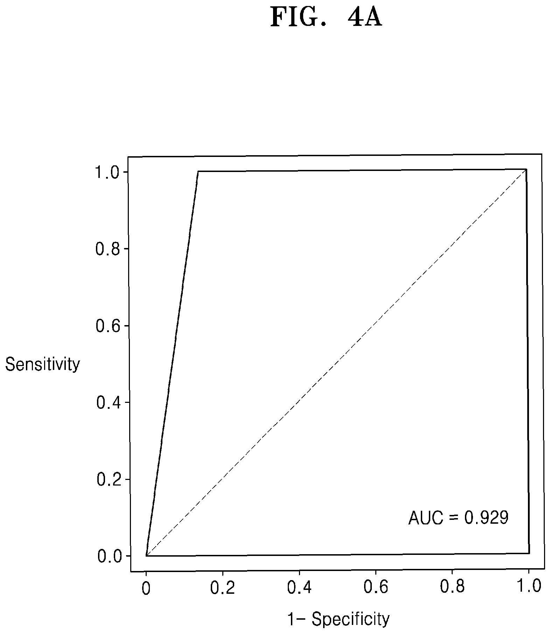

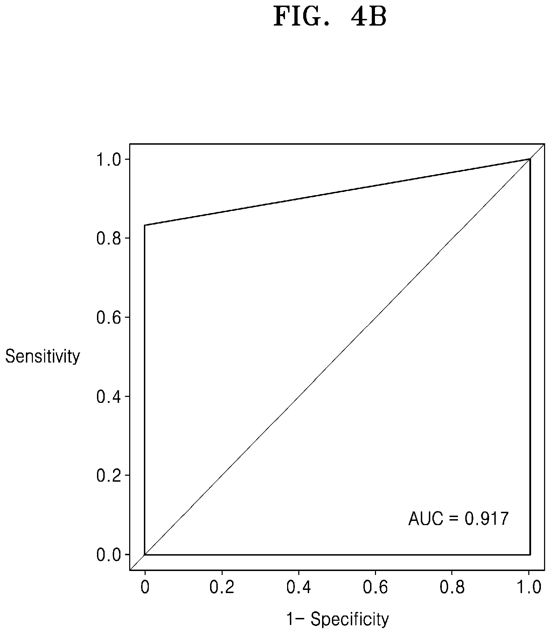

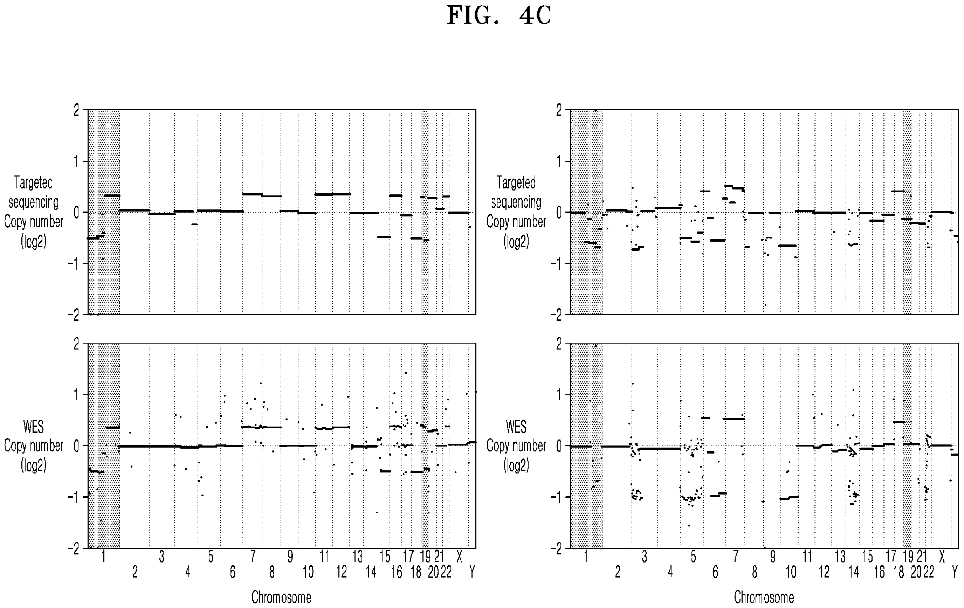

Segmental chromosome levels of 1p and 19q were compared using WES to evaluate whether actual chromosomal co-deletion status is reflected through the genes. When 9 out of 11 genes and 8 out of 9 genes were simultaneously deleted from chromosome arms 1p and 19, respectively, the co-deletion of chromosome levels of 1p and 19q was confirmed in each tumor. ROC curve was identified using 52 specimens to evaluate the accuracy of the selected genes as a biomarker for confirming the 1p/19q co-deletion status, and is shown in FIGS. 4A, 4B, and 4C.

As confirmed in FIG. 4A, it was confirmed that when the gene set and WES results were combined, the 20 biomarker genes accurately predicted the co-deletion of chromosomes 1p and 19q with a high AUC value of 0.929 and a 95 % Cl value of 0.8862-1.

In the results of evaluating whether the target sequencing panel is capable of accurately predicting the co-deletion of 1p/19q compared to conventionally known diagnostic methods such as fluorescence in situ hybridization, as confirmed in FIG. 4B, it was confirmed that the AUC value was 0.917 and the 95 % Cl value was 0.8596-0.9737, showing a high consistent concordance rate through the ROC curve analysis.

As confirmed in FIG. 4C, it was confirmed that both panels showed very similar chromosomal profiles through the results of comparing the co-deletion of chromosomes 1p and 19q derived from WES with the chromosomal deletion predicted from the target sequencing panel.

Therefore, in total, the co-deletion of 1p and 19q, which allow accurate diagnosis or prognosis of glioma was confirmed with high accuracy and sensitivity through the 20 gene biomarkers.

## Claims

1. A composition for diagnosis or prognosis of glioma, the composition comprising:
an agent for detecting variants in at least one gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2.

2. The composition of claim 1, wherein the glioma is at least one selected from the group consisting of astrocytic tumors, oligodendroglial tumors, mixed gliomas, and ependymal tumors.

3. The composition of claim 1, wherein the agent detects co-deletion of gene chromosomes 1p and 19q.

4. The composition of claim 1, wherein in a base sequence, the variants of the genes are:
1) single nucleotide variant;
2) deletion or insertion of a base sequence region of 1 to 50 nucleotides;
3) copy number variant; or
4) a combination of two or more selected from 1) to 3).

5. The composition of claim 1, wherein the agent comprises a primer, a probe, or an antisense nucleotide.

6. A biomarker panel for diagnosis or prognosis of glioma, the biomarker panel comprising:
an agent for measuring variants in at least one gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2.

7. A kit comprising an agent for detecting variants in at least one gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2.

8. A method of providing information related to diagnosis or prognosis of glioma, the method comprising:
1) obtaining a nucleic acid sample from a biological sample of an individual;
2) detecting genetic variants in at least one target gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2 from the obtained sample; and
3) comparing and analyzing the extent of the detected genetic variants with the extent of a normal sample.

9. The method of claim 8, wherein the biological sample is at least one selected from the group consisting of blood, plasma, serum, urine, and saliva of a patient.

10. The method of claim 8, wherein the genetic variants of 2) are:
1) single nucleotide variant;
2) deletion or insertion of a base sequence region of 1 to 50 nucleotides;
3) copy number variant; or
4) a combination of two or more selected from 1) to 3).

11. The method of claim 8, wherein the detecting of 2) is performed through a Next Generation Sequencer (NGS) platform.

12. The method of claim 11, wherein the Next Generation Sequencer platform is whole-genome sequencing, whole-exome sequencing, or target gene panel sequencing.

13. The method of claim 8, wherein the analyzing of 3) is to identify the presence of co-deletion of gene chromosomes 1p and 19q.

14. The method of claim 13, wherein the presence of the co-deletion of gene chromosomes 1p and 19q is determined by detecting copy number variants in at least one gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2.

15. A biomarker panel for personalized medicine for glioma, the biomarker panel comprising an agent for detecting variants in at least one gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2.

16. A method of providing information for personalized treatment, the method comprising:
1) detecting genetic variants in at least one target gene selected from the group consisting of SAMD11, KLHL21, FAM167B, HPCAL4, GPBP1L1, LPHN2, GPR88, ZNF599, C19ORF33, B9D2, BCAM, CABP5, SIGLEC11, ERVV-2, ZNF865, MZF1, MRTO4, LRIG2, BSND, and SLC30A2 from a biological sample isolated from an individual; and
2) setting the individual whose genetic variants are detected in the detection results as a treatment target.
